(19) Europäisches Patentamt / European Patent Office / Office européen des brevets

(11) **EP 3 576 546 B1**

(12) **EUROPEAN PATENT SPECIFICATION**

(45) Date of publication and mention
of the grant of the patent:
**02.08.2023 Bulletin 2023/31**

(21) Application number: **18707754.0**

(22) Date of filing: **02.02.2018**

(51) International Patent Classification (IPC):
*A23D 9/02* *(2006.01)*    *A23L 33/115* *(2016.01)*
*A23L 33/10* *(2016.01)*    *A23L 33/135* *(2016.01)*
*A23L 29/10* *(2016.01)*    *A23D 9/007* *(2006.01)*
*C12N 1/20* *(2006.01)*    *A61K 9/10* *(2006.01)*
*A61K 47/44* *(2017.01)*    *A61K 35/741* *(2015.01)*
*A61K 35/744* *(2015.01)*    *A61K 35/747* *(2015.01)*

(52) Cooperative Patent Classification (CPC):
(C-Sets available)
**A61K 9/10; A23D 9/007; A23D 9/02; A23L 29/10;
A23L 33/10; A23L 33/115; A23L 33/135;
A61K 35/741; A61K 35/744; A61K 35/747;
A61K 47/44; C12N 1/20;** A23V 2002/00    (Cont.)

(86) International application number:
**PCT/IB2018/050677**

(87) International publication number:
**WO 2018/142346 (09.08.2018 Gazette 2018/32)**

(54) **PROBIOTICAL COMPOSITION**

PROBIOTISCHE ZUSAMMENSETZUNG

COMPOSITION PROBIOTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **02.02.2017 IT 201700011632 U**

(43) Date of publication of application:
**11.12.2019 Bulletin 2019/50**

(73) Proprietor: **Farmaceutici Procemsa S.p.A.
10042 Nichelino (IT)**

(72) Inventor: **URAS, Giovanni
10042 Nichelino (IT)**

(74) Representative: **Casciano, Lidia Giulia Rita et al
Studio Torta S.p.A.
Via Viotti, 9
10121 Torino (IT)**

(56) References cited:
**EP-A1- 3 498 101      EP-A1- 3 562 478
WO-A1-03/043432      WO-A1-2016/030440
CN-A- 105 707 897      DE-A1-102011 009 798
US-A1- 2012 058 095      US-A1- 2012 058 225
US-A1- 2012 076 895      US-A1- 2013 251 792**

(52) Cooperative Patent Classification (CPC): (Cont.)

C-Sets
A23V 2002/00, A23V 2200/32, A23V 2200/3204,
A23V 2250/184, A23V 2250/1944

**Description**

TECHNICAL FIELD

The present invention concerns probiotical suspensions.

BACKGROUND ART

Probiotical formulations, for example lactobacilli, are normally sold in the form of suspensions to be reconstituted at the moment of use or alternatively in the form of anhydrous suspensions in an oily carrier. These formulations are necessary since probiotics require an anhydrous environment in order to survive. In fact, the presence of water affects the long-term stability, i.e. microbial count, in the formulation.

However, the suspensions are thermodynamically unstable systems, subject to particle sedimentation and re-aggregation. This physical form allows the administration of insoluble solids in a given solvent in more readily bioavailable pharmaceutical formulations.

For preparation of the suspensions, determination of the particle dimensions is very important in order to limit sedimentation.

In particular, according to the Stokes model, the larger particles deposit sediment more quickly, but can be easily redispersed by stirring; the smaller particles sediment more slowly, but are prone to caking.

One of the causes of instability of dispersed systems like suspensions is due to the action of gravity, which tends to separate the phases that compose the system. Stokes's law correlates the separation velocity of the molecules that constitute the two phases with the parameters that define the physical state of the dispersed system.

$$v = \frac{2\,R^2\,(d_e - d_i)g}{9\eta} \qquad\qquad (1)$$

in which:

$v$ = sedimentation velocity;
$R$ = radius of dispersed particles;
$d_i$ = density of internal phase;
$d_e$ = density of external phase;
$g$ = gravitational acceleration;
$\eta$ = viscosity of continuous phase.

From this formula it can be deduced that the system will be all the more stable the closer the densities of the two phases and the greater the viscosity of the continuous phase.

The dimensions of the particles are also important: the smaller they are, the lower the sedimentation velocity; however, excessively small dimension entails a significant increase in the overall specific surface of the dispersed mass and therefore an increase in instability. The very fine subdivision of the particles can also facilitate undesired adsorption phenomena; for these reasons, with the exception of the suspensions to be reconstituted prior to use, the phenomenon of flocculation is exploited. This technique consists in making the particles aggregate as flocs, able to retain within them a part of solvent. The flocs precipitate more rapidly than the particles, but since they constitute a fairly voluminous sediment, they can be easily redispersed. On the other hand, it is important that the sedimentation velocity of the flocs is such as to guarantee a homogeneous suspension at least for the time necessary for withdrawal of the dose to be administered; this can be easily obtained with appropriate thickening agents ensuring that they are present in quantities such as to permit stirring and flux of the suspension, at the operating temperatures typical for the desired application.

A further factor which, as far as possible, can be taken into consideration, is the choice of solid-solvent combinations with similar density. In this case, as resulting from the Stokes model, the sedimentation velocity tends to be zero.

The known probiotical formulations are commonly suspensions in an oily carrier, for example maize (corn) oil, olive oil or sunflower oil or triglycerides. Probiotical formulations are for example known from WO2016030440, CN105707897, DE102011009798, US2013251792 and US2012076895.

Said formulations are for example suitable for the treatment of infantile colic.

However, also in these formulations the probiotics precipitate, forming an indissoluble cake. Therefore, the end product lacks homogeneity, with problems of uniformity of dosage and therefore of effectiveness.

Furthermore, the stringent laws in the field of paediatrics limit the addition of excipients such as mono and diglycerides of alimentary fatty acids and prevent the use of other lipophilic thickeners, like silicas.

The concentrations permitted in the case of products intended for infancy for these excipients are in fact too low to allow a stable suspension.

DISCLOSURE OF INVENTION

The object of the present invention is therefore to provide a probiotical suspension without the drawbacks listed above.

Said object is achieved by the present invention relative to a composition according to claim 1.

BEST MODE FOR CARRYING OUT THE INVENTION

According to a first aspect of the invention, an oral composition is provided in the form of an anhydrous suspension of probiotics in an oily carrier comprising at least one vegetable fat with a saturated fatty acid content of at least 25% by weight of the weight of the vegetable fat and therefore solid at ambient temperature ,wherein said oily carrier is present in a quantity ranging from 75 to 99% by weight of the total weight of the suspension; and said vegetable fat is present in a quantity ranging from 0.5 to 10% by weight of the total weight of the suspension.

Advantageously, the presence of the vegetable fat solid at ambient temperature allows thickening of the continuous phase of the suspension, overcoming the technical limit not possible with the excipients currently permitted and limited by the current law, while maintaining the visual characteristics and simplicity of use of a normal oily suspension.

In the present text, by "probiotic" we mean a live non-pathogenic organism which, administered in an adequate quantity, is beneficial for the health of the host. Examples of probiotics useful for the present invention include lactic ferment and bacteria, such as lactobacilli, bifidobacteria, streptococci, lactococci, propionylbacteria, and sporogenes and yeasts, such as yeasts of the genus Saccharomyces like Saccharomyces boulardii.

In the present text, by "oily carrier" we mean a liquid anhydrous oily means based on oils, vegetable fats and derivatives.

In the suspension according to the invention the oily carrier is selected from the group consisting of at least one triglyceride of saturated fatty acids having an alkyl chain comprising between 6 and 12 carbon atoms, more preferably between 8 and 10 carbon atoms, vegetable oils and mixtures thereof. Preferably the vegetable oils are selected from the group consisting of maize oil, olive oil, sunflower oil and mixtures thereof. They have been selected for their resistance to lipid peroxidation which improves conservation of the product and in particular minimizes the formation of oxidation products which could compromise the vitality of the enzymes. In the suspension of the invention, the oily carrier is present in a quantity between 75 and 99%, preferably between 80 and 95% by weight of the total weight of the suspension.

According to the invention, the vegetable fat can be selected from the group consisting of shea butter, cocoa butter, palm oil, palm butter, shorea butter, coconut oil, coconut butter, also partially or totally hydrogenated. The particular fatty acid composition of these vegetable fats and in particular the saturated portion results in the formation of a slightly gelified solution in direct proportion to the concentration added and to the ambient temperature.

In the suspension of the invention the vegetable fat is present in a quantity ranging from 0.5 to 10%, preferably from 2 to 5% by weight of the total weight of the suspension.

The suspension can further contain one or more emulsifiers selected from the group consisting of E304 (ascorbyl palmitate), E322 (lecithin), E471 (mono and diglycerides of fatty acids), E472c (citric esters of fatty acids), E473 (sucrose esters of fatty acids). The mixture for example has the function of further contributing to achieving the desired viscosity for the oily phase and of improving the dispersion in water of the preparation during the phase of mixing in water.

The solution provided by the invention is particularly suited to the suspension of probiotics selected from the group consisting of the genus Lactobacillus, such as for example L. acidophilus, L. rhamnosus, L. reuteri; and of the genus Bifidobacterium such as B. infantis, B. breve, B. longum.

Further characteristics of the present invention will become evident from the following description of some merely illustrative and non-limiting examples.

Example 1

A suspension was prepared having the composition shown in table 1.

Table 1

| Raw material | % w/w (100 grams) |
|---|---|
| MEDIUM-CHAIN TRIGLYCERIDES (NEOBEE; others) | 85.000 |
| E471 (COMPRITOL ATO) | 0.4 |

(continued)

| Raw material | % w/w (100 grams) |
| --- | --- |
| SHEA BUTTER | 2.5 |
| LACTOBACILLUS RHAMNOSUS | 12.1 |

The suspension is prepared by initially heating the medium-chain triglycerides to 65°C under constant stirring in the primary melter. The E471 is then added and mixed until it has completely dissolved and a uniform, clear and transparent mixture is obtained.

The mixture is then slowly and gradually cooled in a water bath under constant stirring; once 50°C is reached, the shea butter is added until perfectly dissolved, the solution is transparent. Cooling is continued, preferably in a cold water bath, and once the temperature of 20°C has been reached, the solution appears slightly opalescent; the probiotic is added, carefully homogenizing to avoid all lumps, preferably in a modified atmosphere until a homogeneous suspension is obtained.

**Claims**

1. An oral composition in the form of an anhydrous suspension of probiotics in an oily carrier comprising at least one vegetable fat that is solid at ambient temperature and has a saturated fatty acid content of at least 25% by weight of the weight of the vegetable fat wherein

   said oily carrier is present in a quantity ranging from 75 to 99% by weight of the total weight of the suspension; and
   said vegetable fat is present in a quantity ranging from 0.5 to 10% by weight of the total weight of the suspension.

2. The composition according to claim 1, **characterized in that** said probiotics are selected from the group consisting of lactic ferment and yeasts.

3. The composition according to claim 1 or 2, **characterized in that** said probiotics are selected from the group consisting of lactobacilli, bifidobacteria, streptococci, lactococci, propionyl bacteria, sporogenes and yeasts of the genus Saccharomyces.

4. The composition according to claim 1, **characterized in that** said oily carrier is selected from the group consisting of at least one triglyceride of saturated fatty acids having an alkyl chain comprising between 6 and 12 atoms of carbon, vegetable oils and mixtures thereof.

5. The composition according to claim 4, **characterized in that** said vegetable oils are selected from the group consisting of maize oil, olive oil, sunflower oil and mixtures thereof.

6. The composition according to claim 4, **characterized in that** said triglyceride is at least one triglyceride of saturated fatty acids having an alkyl chain comprising between 8 and 10 atoms of carbon.

7. The composition according to claim 1, **characterized in that** said oily carrier is present in a quantity ranging from 80 to 95% by weight of the total weight of the suspension.

8. The composition according to claim 1, **characterized in that** said vegetable fat is selected from the group consisting of shea butter, cocoa butter, palm oil, palm butter, shorea butter, coconut oil and coconut butter.

9. The composition according to claim 1, **characterized in that** said vegetable fat is present in a quantity ranging from 2 to 5% by weight of the total weight of the suspension.

10. The composition according to claim 1, **characterized in that** it further comprises one or more emulsifiers selected from the group consisting of E304 (ascorbyl palmitate), E322 (lecithin), E471 (mono- and diglycerides of fatty acids), E472c (citric esters of fatty acids) and E473 (sucrose esters of fatty acids).

**Patentansprüche**

1.  Orale Zusammensetzung in der Form einer wasserfreien Suspension von Probiotika in einem öligen Träger, umfassend mindestens ein pflanzliches Fett, das bei Raumtemperatur fest ist und einen Gehalt an gesättigten Fettsäuren von mindestens 25 Gew.-% bezogen auf das Gewicht des pflanzlichen Fettes hat, wobei

    der ölige Träger in einer Menge von 75 bis 99 Gew.-% bezogen auf das Gesamtgewicht der Suspension vorhanden ist; und
    das pflanzliche Fett in einer Menge von 0,5 bis 10 Gew.-% bezogen auf das Gesamtgewicht der Suspension vorhanden ist.

2.  Zusammensetzung nach Anspruch 1, **dadurch gekennzeichnet, dass** die Probiotika ausgewählt sind aus der Gruppe bestehend aus Milchsäureferment und Hefen.

3.  Zusammensetzung nach Anspruch 1 oder 2, **dadurch gekennzeichnet, dass** die Probiotika ausgewählt sind aus der Gruppe bestehend aus Laktobazillen, Bifidobakterien, Streptokokken, Laktokokken, Propionylbakterien, Sporogene und Hefen der Gattung Saccharomyces.

4.  Zusammensetzung nach Anspruch 1, **dadurch gekennzeichnet, dass** der ölige Träger ausgewählt ist aus der Gruppe bestehend aus mindestens einem Triglycerid von gesättigten Fettsäuren mit einer alkyl-Kette umfassend zwischen 6 und 12 Kohlenstoffatome, pflanzlichen Ölen und Mischungen davon.

5.  Zusammensetzung nach Anspruch 4, **dadurch gekennzeichnet, dass** die pflanzlichen Öle ausgewählt sind aus der Gruppe bestehend aus Maisöl, Olivenöl, Sonnenblumenöl und Mischungen davon.

6.  Zusammensetzung nach Anspruch 4, **dadurch gekennzeichnet, dass** das Triglycerid mindestens ein Triglycerid von gesättigten Fettsäuren mit einer Alkylkette umfassend zwischen 8 bis 10 Kohlenstoffatomen ist.

7.  Zusammensetzung nach Anspruch 1, **dadurch gekennzeichnet, dass** der ölige Träger in einer Menge von 80 bis 95 Gew.-% bezogen auf das Gesamtgewicht der Suspension vorhanden ist.

8.  Zusammensetzung nach Anspruch 1, **dadurch gekennzeichnet, dass** das pflanzliche Fett ausgewählt ist aus der Gruppe bestehend aus **Sheabutter**, Kakaobutter, Palmöl, Palmbutter, Shoreabutter, Kokosöl und Kokosbutter.

9.  Zusammensetzung nach Anspruch 1, **dadurch gekennzeichnet, dass** das pflanzliche Fett in einer Menge von 2 bis 5 Gew.-% bezogen auf das Gesamtgewicht der Suspension vorhanden ist.

10. Zusammensetzung nach Anspruch 1, **dadurch gekennzeichnet, dass** sie weiterhin einen oder mehrere Emulgatoren ausgewählt aus der Gruppe **bestehend aus** E304 (Ascorbylpalmitat), E322 (Lecithin), E471 (Mono- und Diglyceride von Fettsäuren), E472c (Zitronensäureester von Fettsäuren) und E473 (Saccharoseester von Fettsäuren) umfasst.

**Revendications**

1.  Composition orale sous la forme d'une suspension anhydre de probiotiques dans un vecteur huileux comprenant au moins une matière grasse végétale qui est solide à température ambiante et présente une teneur en acides gras saturés d'au moins 25 % en poids du poids de la matière grasse végétale dans laquelle

    ledit vecteur huileux est présent en une quantité allant de 75 à 99 % en poids du poids total de la suspension ; et
    ladite matière grasse végétale est présente en une quantité allant de 0,5 à 10 % en poids du poids total de la suspension.

2.  Composition selon la revendication 1, **caractérisée en ce que** lesdits probiotiques sont sélectionnés à partir du groupe consistant en ferment lactique et levures.

3.  Composition selon la revendication 1 ou 2, **caractérisée en ce que** lesdits probiotiques sont sélectionnés à partir du groupe consistant en lactobacilles, bifidobactéries, streptocoques, lactocoques, bactéries de propionyle, sporogènes et levures du genre Saccharomyces.

4. Composition selon la revendication 1, **caractérisée en ce que** ledit vecteur huileux est sélectionné à partir du groupe consistant en au moins un triglycéride d'acides gras saturés présentant une chaîne alkyle comprenant entre 6 et 12 atomes de carbone, des huiles végétales et des mélanges de ceux-ci.

5. Composition selon la revendication 4, **caractérisée en ce que** lesdites huiles végétales sont sélectionnées à partir du groupe consistant en huile de maïs, huile d'olive, huile de tournesol et des mélanges de celles-ci.

6. Composition selon la revendication 4, **caractérisée en ce que** ledit triglycéride est au moins un triglycéride d'acides gras saturés présentant une chaîne alkyle comprenant entre 8 et 10 atomes de carbone.

7. Composition selon la revendication 1, **caractérisée en ce que** ledit vecteur huileux est présent en une quantité allant de 80 à 95 % en poids du poids total de la suspension.

8. Composition selon la revendication 1, **caractérisée en ce que** ladite huile végétale est sélectionnée à partir du groupe consistant en beurre de karité, beurre de cacao, huile de palme, beurre de palme, beurre de Shorea, huile de noix de coco et beurre de noix de coco.

9. Composition selon la revendication 1, **caractérisée en ce que** ladite huile végétale est présente en une quantité allant de 2 à 5 % en poids du poids total de la suspension.

10. Composition selon la revendication 1, **caractérisée en ce qu'**elle comprend en outre un ou plusieurs émulsifiants sélectionnés à partir du groupe consistant en E304 (palmitate d'ascorbyle), E322 (lécithine), E471 (monoglycérides et diglycérides d'acides gras), E472c (esters citriques d'acides gras) et E473 (esters de sucrose d'acides gras).

**REFERENCES CITED IN THE DESCRIPTION**

**Patent documents cited in the description**

- WO 2016030440 A
- CN 105707897
- DE 102011009798
- US 2013251792 A
- US 2012076895 A